# EUROPÄISCHE PATENTSCHRIFT

(11) **EP 0 940 383 B1**
(45) Veröffentlichungstag und Bekanntmachung des Hinweises auf die Patenterteilung: **18.06.2003**
(21) Anmeldenummer: 99103350.7
(22) Anmeldetag: 20.02.1999
(51) Int. Cl.: C07C 29/40, C07C 67/29, C07B 53/00, C07F 3/06

(54) **Verfahren zur selektiven Alkylierung von Aldehyden mit Organozinkverbindungen**
Method for the selective alkylation of aldehydes with organozinc compounds
Méthode d'alkylation sélective d'aldéhydes avec composés organozinc

(30) Priorität: 05.03.1998 DE 19809341
(43) Veröffentlichungstag der Anmeldung: 08.09.1999
(73) Patentinhaber: Chemetall GmbH, 60487 Frankfurt (DE); PPG - SIPSY, 49242 Avrillé cedex (FR)
(72) Erfinder: Knochel, Paul Prof. Dr., 35037 Marburg (DE); Lutz, Christian Dr., 79576 Weil am Rhein (DE)
(74) Vertreter: Uppena, Franz, Dr.

(56) Entgegenhaltungen:
- S. NIWA: "Catalytic asymmetric synthesis of optically active alkynyl alcohols by enantioselective alkynylation of aldehydes and by enantioselective alkylation of alkynyl aldehydes" JOURNAL OF THE CHEMICAL SOCIETY, PERKIN TRANSACTIONS 1,1990, Seiten 937-943, XP002121985 LETCHWORTH GB
- C. LUTZ: "Highly enantioselective addition of mixed diorganozincs to aldehydes" JOURNAL OF ORGANIC CHEMISTRY, Bd. 62, Nr. 22, 31. Oktober 1997 (1997-10-31), Seiten 7895-7898, XP002121986 EASTON US
- H. TAKAHASHI: "A catalytic enantioselective reaction using a C2-symmetric disulfonamide as a chiral ligand: alkylation of aldehydes catalyzed by disulfonamide-Ti(O-i-Pr)4-diakyl zinc system" TETRAHEDRON, (INCL TETRAHEDRON REPORTS), Bd. 48, Nr. 27, 3. Juli 1992 (1992-07-03), Seiten 5691-5700, XP002121987 OXFORD GB

## Beschreibung

Gegenstand der Erfindung ist ein Verfahren zur selektiven Alkylierung von Aldehyden mittels Organozinkverbindungen.

Organozinkverbindungen R-Zn-X bzw. R₂Zn (R = Alkyl, Aryl, X = Cl, Br, I) sind gut handhabbare organometallische Reagenzien, die in der Synthese für selektive CC-Verknüpfungsreaktionen genutzt werden können, wobei es aufgrund der geringen Reaktivität der Zinkspezies für die Übertragung des Restes R der Anwesenheit einer weiteren Übergangsmetallverbindung bedarf, z.B. einer Kupfer-, Palladium-, Kobalt- oder Titanverbindung. Ein besonderer Vorteil der zinkorganischen Chemie liegt darin begründet, daß die o.a. organischen Reste R auch eine funktionelle Gruppe FG aufweisen können, wie z.B. eine Ester-, Amin- oder Nitrilfunktion. (Einen guten Überblick geben P. Knochel und R.D. Singer in Chem. Rev. 1993, 93, 2117 - 2188.)

Von besonderer Bedeutung ist die Übertragung funktionalisierter organischer Reste (R-FG) auf Aldehyde, da diese Reaktion in Anwesenheit geeigneter chiraler Hilfsstoffe (Liganden) enantioselektiv verläuft:

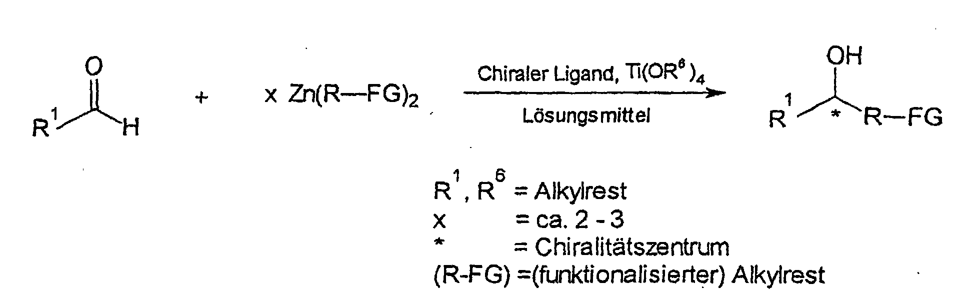

Auf diese Art können funktionalisierte sekundäre Alkohole mit in der Regel guten Ausbeuten und sehr guten Enantiomerenüberschüssen (> 90%) hergestellt werden. Diese exzellente Selektivität wird durch eine relativ schlechte Reaktivität der Zn-Spezies erkauft. Letztere hat zur Folge, daß die Zinkverbindung im Regelfall mit 2-3fachem Überschuß eingesetzt werden muß, d.h. von den eingesetzten Mengen (R-FG) werden letztendlich nur ca. 15 bis 30% auf den Aldehyd übertragen. Der Rest geht bei der Aufarbeitung verloren. Ähnliches gilt für die als co-Reagenz eingesetzte Titanverbindung, die vorzugsweise in etwa equimolaren Mengen (bezogen auf die Zn-Verbindung) eingesetzt werden muß.

Um die Ausnutzung des zinkgebundenen Alkylrestes zu erhöhen, wurden jüngst gemischte Zinkverbindungen vom Typ

(FG-R)-Zn-CH₂Si(CH₃)₃

eingesetzt [C. Lutz, P. Knochel, J. Org. Chem. 1997, 62, 7895]. Es wurde gefunden, daß der H₂C-Si(CH₃)₃-Rest ("TMSM") nicht auf Carbonylverbindungen übertragen wird und somit eine gewünschte chemoselektive Übertragung des funktionalisierten Alkylrestes (R-FG) gewährleistet ist.

Auf diese Weise wird der zu übertragende Rest (R-FG) in bedeutend niedrigeren Mengen benötigt. Dies ist vor allem für wertvolle funktionalisierte Reste von hohem Interesse. Für technische Anwendungen ist der TMSM-Rest jedoch wegen mangelnder Verfügbarkeit und des hohen Preises seiner Vorläuferverbindung Cl-CH₂-Si(CH₃)₃ wenig geeignet.

Aufgabe der Erfindung ist es daher, die aus dem Stand der Technik bekannten Nachteile zu vermeiden und insbesondere ein Verfahren zur (enantioselektiven) Alkylierung von Aldehyden mittels zinkorganischer Verbindungen zu schaffen, bei dem der zu übertragende Alkylrest funktionalisiert vorliegen kann und jeweils nur eine Valenz des Zinks absättigt, und die zweite Zinkbindung durch einen die Alkylierung nicht störenden, gut zugänglichen und preiswerten Rest ("Dummy-Ligand") abgesättigt wird.

Weitere Aufgabe der Erfindung ist es, neue Verbindungen zu schaffen, mit denen sich die (enantioselektive) Alkylierung von Aldehyden entsprechend dem erfindungsgemäßen Verfahren besonders vorteilhaft durchführen läßt.

Trotz des in der Literatur beschriebenen Vorurteils [Steven H. Bertz et al., J. Am. Chem. Soc. 1996, 118, 10906], welches postuliert, daß es zur Stabilisierung der Zn-Dummy-Bindung (und damit der Nichtübertragung des Dummy-Liganden) wie beim o.a. TMSM-Rest eines Si-Atoms in β-Stellung zum Zn-Atom bedarf (β-Silyleffekt zu Metallzentren), wurde überraschend gefunden, daß auch eine Reihe einfacher, siliziumfreier, organischer Reste vom Typ 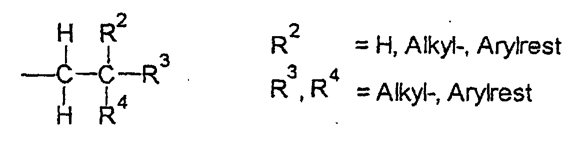 oder 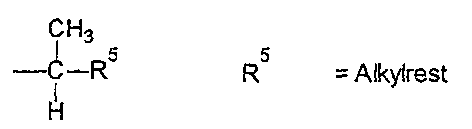 ebenfalls in der Lage sind, als Dummy-Liganden in den oben beschriebenen zinkorganischen Verbindungen zu fungieren. Bevorzugt eignen sich Alkyleste mit 1 bis 8 C-Atomen und Phenylreste, die wahlweise zusätzlich einen oder mehrere Alkylsubstituenten tragen können, insbesondere Dummy-Liganden mit R² = Phenyl und R³, R⁴ = Methyl), bzw. mit R², R³, R⁴ = Methyl. Die wesentlichen Merkmale des erfindungsgemäßen Verfahrens sind im Patentanspruch 1 angegeben. Die Unteransprüche 2 bis 10 offenbaren Merkmale, die das erfindungsgemäße Verfahren weiterbilden. Die Ansprüche 11 und 12 beziehen sich auf eine neue Gruppe von Dialkylzinkverbindungen, mit denen die chemoselektive Übertragung von (funktionalisierten) Alkylresten nach dem erfindungsgemäßen Verfahren besonders gut verläuft.

Zur Durchführung des erfindungsgemäßen Verfahrens können grundsätzlich alle Aldehyde und insbesondere folgende Aldehyde eingesetzt werden: gesättigte Aldehyde mit n-Alkylketten und verzweigten Alkylketten, aromatische und heteroaromatische Aldehyde, Benzaldehyd, substituierter Benzaldehyd, wie z.B. Anisaldehyd, Pyridin-2-carbaldehyd, Pyridin-3-carbaldehyd, Pyridin-4-carbaldehyd, Chinolin-3-carbaldehyd, Chinolin-4-carbaldehyd, Isochinolin-4-carbaldehyd und weitere heteroaromatische Aldehyde, ungesättigte Aldehyde, Zimtaldehyd und Derivate, Alkenale, 1-Cyclopenten-1-carbaldehyd, 1-Cyclohexen-1-carbaldehyd, Alkinale, funktionalisierte Aldehyde, Aminoaldehyde und Hydroxyaldehyde.

Als Reaktion für die (enantioselektive) Alkylierung von Aldehyden mittels solcher zinkorganischer Verbindungen, deren einer Ligand aus dem chemoselektiv zu übertragenden (funktionalisierten) Rest (R-FG) und deren anderer Ligand aus einem gut zugänglichen und preiswerten Dummy besteht, wurde folgende Umsetzung gewählt:

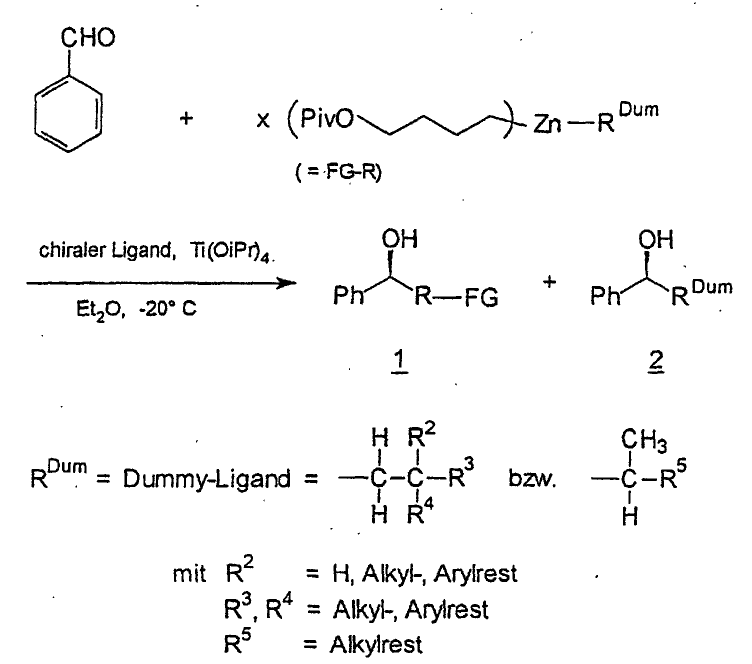

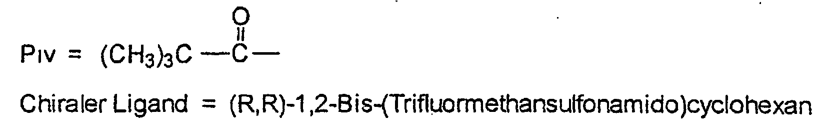

| | x | Ausbeute isoliert | **1 : 2** ^{**1)**} | ee 1 ²⁾ |
|---|---|---|---|---|
| R², R³, R⁴ = Me (Neopentyl) | 2.4 | 82 % | **100 : 0** | 89 % |
| R², R³ = Me, R⁴=Ph (Neophyl) | 2.4 | 82% | **100 : 0** | 89 % |
| R², R³ = Me, R⁴ = H (i-Bu) | 2.4 | ---- | **95 : 5** | 94 % |
| R⁵ = Me (i-Pr) | 2.4 | ---- | **82 : 18** | rac. |

| | | | | |
|---|---|---|---|---|
| ¹⁾ Bestimmt durch a) GC-Säule und b) HPLC- Analyse; a) Chrompack Chirasil CB, Trägergas H₂, 100 kPa; b) Chiracel OD, UV- Detektor, 215 nm. | | | | |
| ²⁾ Bestimmt durch chirale HPLC-Analyse, Chiracel OD. ee = prozentualer Überschuß des Enantiomers gegenüber dem Racemat. | | | | |

Das Katalysatorsystem für die Addition von gemischten (teils funktionalisierten) Dialkylzinkverbindungen an Aldehyde besteht hier aus (R,R)-1,2-Bis-(Trifluormethansulfonamido)cyclohexan als chiralem Ligand und Titantetraisopropylat als Lewissäure. Wie aus den in der o.a. Tabelle angegebenen Ergebnissen der Testreaktion ersichtlich ist, wird im Falle des Neopentyl-(Dummy)-Liganden (R², R³, R⁴ = Me) und des Neophyl-(Dummy)-Liganden (R², R³ = Me, R⁴ = Ph) nur der gewünschte Rest (R-FG), hier 4-Pivaloxybutyl, auf den Aldehyd übertragen, und zwar weitgehend enantioselektiv. Ein Wechsel zu sterisch weniger anspruchsvollen Dummy-Liganden, wie Isobutyl (R², R³ = Me, R⁴ = H), und Isopropyl (R⁵ = Me), führt hier zu einer partiellen Übertragung des Dummy-Liganden auf den Aldehyd (5% bzw. 18%). Gleichwohl wird das gewünschte Reaktionsprodukt 1 immer noch in deutlichem Überschuß zum nicht gewünschten Produkt 2 erhalten.

Damit ist gezeigt, daß mit dem erfindungsgemäßen Verfahren chemoselektiv der zu übertragende Rest (R-FG) von der Dialkylzinkverbindung auf den Aldehyd übertragen wird, wobei der Dummy-Ligand der Dialkylzinkverbindung aus gut zugänglichen und preiswerten organischen Resten besteht. Weiterhin können auch mit diesem neuen Verfahren in der Regel gute Enantiomerenüberschüsse erzielt werden.

Besteht der zu übertragende Rest (R-FG) aus einfachen, nicht funktionalisierten Alkylresten (C₁ - C₁₂), so kann die Zugabe des gemischten Dialkylzinkreagenzes von x = 2.4 auf x = 1.6 Äquivalente reduziert werden. Diese Dialkylzinkreagenzien sind in der Regel reaktiver als Dialkylzinkverbindungen, in denen (R-FG) ein funktionalisierter Alkylrest ist.

Zur Durchführung des erfindungsgemäßen Verfahrens werden 1 bis 40 mol%, bevorzugt 5 bis 20 mol%, (bezogen auf den Aldehyd) eines chiralen Liganden, (z.B. (R,R)-1,2-Bis-(Trifluormethansulfonamido)cyclohexan oder (S,S)-1,2-Bis-(Trifluormethansulfonamido)cyclohexan) in einem Lösungsmittel, wie einem Kohlenwasserstoff (C₅-C₁₂), einem aromatischen Kohlenwasserstoff (bevorzugt Toluol), einem Ether (bevorzugt Diethylether) oder einem chlorierten Kohlenwasserstoff (bevorzugt Dichlormethan) bei Raumtemperatur suspendiert bzw. gelöst. Anschließend wird mit 10 bis 200 mol%, bevorzugt mit 60 bis 120 mol%, (bezogen auf den Aldehyd) Lewissäure, z.B. Titan - oder Zirkontetraalkoxid, bevorzugt Titantetraisopropylat, versetzt.

Diese Katalysatorlösung wird nun abgekühlt (ca. -20°C bis 0°C) und mit 100 bis 300 mol%, bevorzugt mit 160 bis 240 mol%, (bezogen auf den Aldehyd) der gemischten, (evtl. funktionalisierten) Dialkylzinkverbindung versetzt. Es wird eine gelbe Lösung erhalten, die auf die gewünschte Reaktionstemperatur mittels Kryostat temperiert wird. Die Additionsreaktionen können in einem Temperaturbereich von -80°C bis +50°C durchgeführt werden, bevorzugt bei -20°C. Zu Katalysator und Reagenz wird 1 Äquivalent Aldehyd langsam zugegeben. Als Aldehyd können aromatische, heteroaromatische, aliphatische, ungesättigte, und funktionelle Gruppen tragende Aldehyde eingesetzt werden. Funktionelle Gruppen (FG) können z.B. sein: Ester, Amin - und Nitrilfunktionen.

Nach beendeter Reaktion wird entsprechend den physikalischchemischen Eigenschaften der Produkte aufgearbeitet. Häufig wird zunächst mit gesättigter NH₄Cl-Lösung hydrolysiert und der entstandene Niederschlag wird mit wenig 10%iger wässriger Salzsäure gelöst. Alternativ wird die organische Phase mit organischen Säuren und/oder Wasser hydrolysiert. Nach Abtrennung der organischen Phase und wiederholter Extraktion der wässrigen Phase werden die vereinigten organischen Phasen z.B. mit 2N Natronlauge gewaschen und über Trockenmittel wie Magnesiumsulfat oder Natriumsulfat getrocknet. Durch Ansäuern der wässrigen, basischen Phase mit verdünnter Schwefelsäure (pH ca. 2) kann der chirale Ligand (R,R)-1,2-Bis-(Trifluormethansulfonamido)cyclohexan mit Diethylether extrahiert und wiederverwendet werden.

Gemischte Dialkylzinkverbindungen sind prinzipiell bekannt. Da diese Verbindungen am günstigsten aus kommerziell verfügbarem Diethylzink hergestellt werden, handelt es sich dabei überwiegend um Ethylzinkalkyle.

Als neue Gruppe von Dialkylzinkverbindungen, mit der die chemoselektive Übertragung von (funktionalisierten) Alkylresten nach dem erfindungsgemäßen Verfahren besonders gut verläuft, wurden Dialkylzinkverbindungen, die den Neophylrest als Dummy-Liganden enthalten, gefunden. Diese Verbindungen sind bisher nicht bekannt.

Zur Herstellung der gemischten Dialkylzinkreagenzien stehen drei Methoden zur Verfügung:
1. Übertragung des Dummy-Liganden mittels der entsprechenden Alkyllithium- oder Alkylmagnesiumhalogen- bzw. Dialkylmagnesiumverbindung auf das funktionalisierte Alkylzinkhalogenid (Transmetallierung). 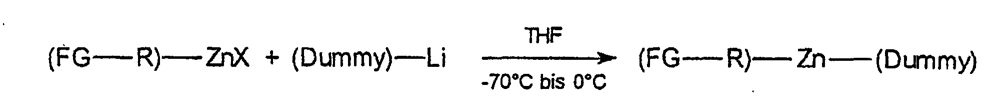 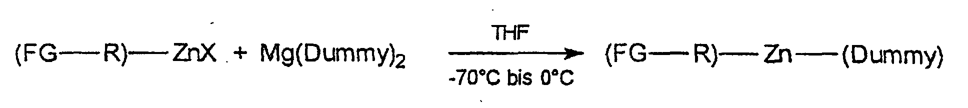 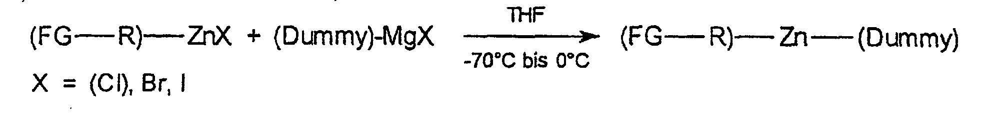 Alkylzinkhalogenide können leicht aus Zink und Alkylhalogenid durch Zinkinsertion in die entsprechende Kohlenstoff-Halogen-Bindung dargestellt werden. Zu beachten ist hier die hohe Toleranz gegenüber anderen funktionellen Gruppen im Alkylrest, wie z.B. Ester, Amin- oder Nitrilfunktionen.
2. Übertragung der (funktionalisierten) Alkyllithium - oder Alkylmagnesiumhalogen- bzw. Dialkylmagnesiumverbindung auf das Alkylzinkhalogenid des Dummy-Liganden (gegenüber 1. umgekehrte Transmetallierung). 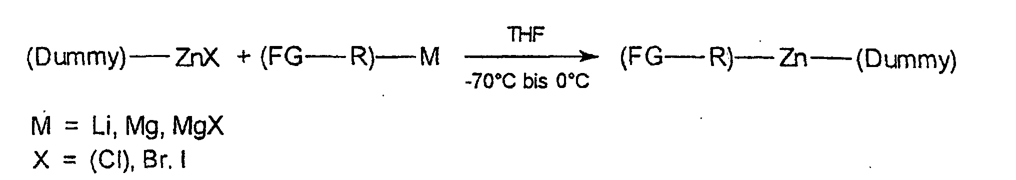
3. Mischen der beiden Dialkylzinkreagenzien (funktionalisierte Dialkylzinkverbindung und Dummy-Dialkylzinkverbindung) zur gemischten Dialkylzinkverbindung.

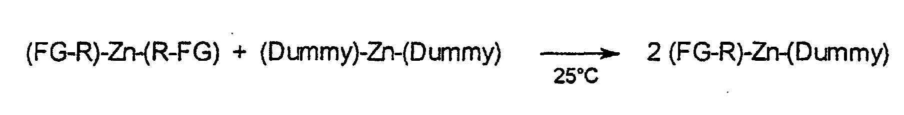

Die Dialkylzinkverbindungen können nach literaturbekannten Methoden erhalten werden. Zur Synthese funktionalisierter Dialkylzinkreagenzien kommen im besonderen der an sich bekannte Iod - Zink - Austausch und der an sich bekannte Bor-Zink - Austausch in Frage.

Methode 3. ist besonders wichtig, da hier keine Salze als Nebenprodukte gebildet werden. Die Gegenwart von Salzen kann die oben beschriebene enantioselektive Addition des (funktionalisierten) Alkylrestes (R-FG) an Aldehyde nachteilig beeinflussen (geringer Umsatz, schlechte Enantioselektivität).

Das beschriebene Verfahren kann auch mit weiteren Katalysatorsystemen (bestehend aus einem chiralen Liganden und evtl. einer Lewissäure) durchgeführt werden. Wie bekannt, gibt es im allgemeinen zwei Klassen von Katalysatorsystemen: 1. chiral modifizierte Lewissäuren und 2. chirale Aminoalkohole.

Folgende weitere Katalysatoren wurden getestet:
1. Chiral modifizierte Lewissäuren: 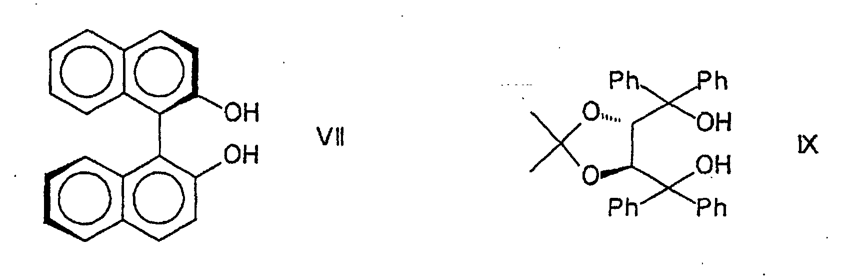 (und jeweils das andere Enantiomer)
2. Aminoalkohole: 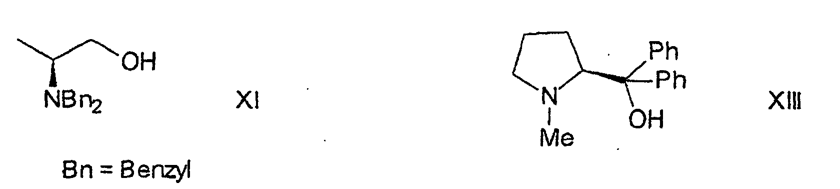 (und jeweils das andere Enantiomer)

Als Reaktion wurde die Addition des Ethylrestes an Benzaldehyd unter Einsatz des Neopentylrestes als Dummy-Ligand gewählt:

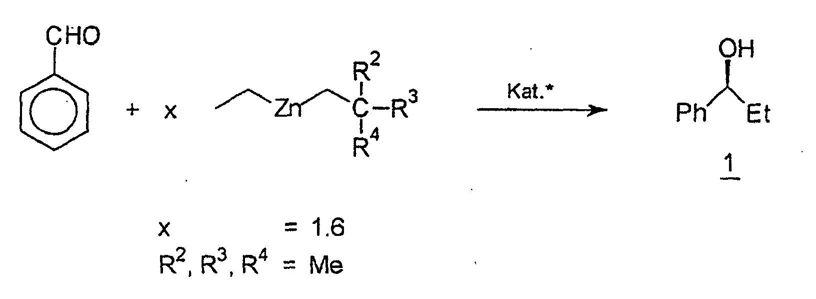

| | Kat.* | X | Ausbeute isoliert | ee 1 ¹⁾ |
|---|---|---|---|---|
| R², R³, R⁴=Me (Neopentyl) | 2 mol % VII + Ti(OiPr)₄, | 1.6 | 92 % | 86 % |
| | Diethylether, | | | |
| | -20°C | | | |
| R², R³, R⁴ = Me (Neopentyl) | 10mol % IX + Ti(OiPr)₄, | 1.6 | 87 % | 79 % |
| | Diethylether | | | |
| | -20°C | | | |
| R², R³, R⁴ = Me (Neopentyl) | 10 mol % XI, | 1.6 | 81 % | 65 % |
| | Toluol, | | | |
| | RT | | | |
| R², R³, R⁴ = Me (Neopentyl) | 2 mol % XIII, | 1.6 | 74 % | 82 % |
| | Toluol, | | | |
| | RT | | | |

| | | | | |
|---|---|---|---|---|
| 1) Bestimmt durch chirale HPLC-Analyse, Chiracel OD. Angaben in mol%, bezogen auf Aldehyd | | | | |

Die Ergebnisse der Testreaktion zeigen, daß chiral modifizierte Lewissäuren oder chirale Aminoalkohole als chirale Liganden (Katalysatoren) im erfindungsgemäßen Verfahren eingesetzt werden können.

Gegenüber den bisher bekannten Methoden erlaubt das erfindungsgemäße Verfahren, selektiv und damit ökonomisch nur den (funktionalisierten) Rest (R-FG) einer Dialkylzinkverbindung auf Aldehyde zu übertragen, d.h. anstelle von bisher nur ca. 15 bis 30% der eingesetzten Menge (R-FG) können jetzt ca. 30 bis 50% übertragen werden. Dabei können vorteilhafterweise hohe Enantiomerenüberschüsse erzielt werden, insbesondere bei Einsatz der erfindungsgemäßen Dialkylzinkverbindungen mit dem Neophylrest als Dummy-Ligand.

Bei den Dummy-Liganden kann auf kommerziell verfügbare, preiswerte Rohstoffe zurückgegriffen werden. Weiterhin läßt sich die Menge an zuzugebender Lewissäure (co-Katalysator) von bisher 2 bis 3 Äquivalenten auf bevorzugt ca. 0.6 bis 1.2 Äquivalente reduzieren, bzw. es lassen sich auch andere Katalysatorsysteme einsetzen.

### Ausführungsbeispiele:

Die Beispiele 1 und 2 haben die Synthese von (S)-5-Hydroxy-5-phenylpentylpivalat mittels des erfindungsgemäßen Verfahrens zum Inhalt, in Beispiel 1 wurde der Neophylrest, in Beispiel 2 der Neopentylrest als Dummy-Ligand verwendet.

Die Beispiele 3 bis 5 beschreiben die Synthese von (S)-1-Phenylethanol, wobei in Beispiel 3 nach bisherigem Stand der Technik gearbeitet wurde und zum Vergleich in den Beispielen 4 und 5 wieder das erfindungsgemäße Verfahren angewandt wurde.

### Beispiel 1: Synthese von (S)-5-Hydroxy-5-phenylpentylpivalat durch enantioselektive Addition der 4-Pivaloxybutylgruppe an Benzaldehyd unter Zuhilfenahme der entsprechenden Neophylzinkverbindung und Darstellung der Neophylzinkverbindung

### Darstellung von Neophylzinkiodid:

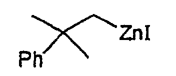

In einem im Vakuum ausgeheizten und mit Argon gespülten Rundkolben mit Stickstoffansatz, Innenthermometer und Septumkappe wurde Zink-Pulver (1.57 g, 24.0 mmol) mit THF (6 ml) überschichtet, vorgelegt und 1,2-Dibromethan (0.2 ml) langsam aus einer Einwegspritze mit Kanüle zugetropft. Nach Zugabe wurde mit einem Heißluftfön bis zum leichten Sieden des Lösungsmittels erwärmt. Nachdem das Reaktionsgemisch auf Raumtemperatur abgekühlt war, wurde Trimethylsilylchlorid (0.2 ml) langsam zugetropft. Das Gemisch wurde erneut mit einem Heißluftfön zum leichten Sieden erwärmt und 5 min. nachgerührt. Anschließend wurde 1-(2-Iod-1,1-dimethylethyl)benzen (Neophyliodid, 1.56 g, 6.0 mmol) innerhalb von 5 min. zugegeben. Nach beendeter Zugabe wurde 3 h auf 50°C erwärmt. Die Zinkinsertion wurde durch Iodolyse und Hydrolyse eines Aliquots der Reaktionsmischung durch GC-Analyse verfolgt.

### Darstellung von Dineophylzink:

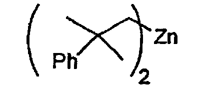

In einem ausgeheizten, mit Argon gespülten 250 ml Dreihals-Rundkolben mit Rückflußkühler, Tropftrichter und Stickstoffhahn wurde Magnesium (4.0 g, 165 mmol) mit Diethylether (25 ml) überschichtet und mit wenigen Körnern Iod aktiviert. 1-(2-Chlor-1,1-dimethyl)benzen (Neophylchlorid, 25.29 g, 150 mmol), gelöst in Diethylether (100 ml), wurde langsam zugetropft. Während der Zugabe wurde das Reaktionsgemisch zum leichten Sieden erhitzt und nach beendeter Zugabe noch 1.5 h refluxiert. In einem weiteren Rundkolben mit Stickstoffansatz wurde Zinkbromid (16.9 g, 75 mmol) im Vakuum (0.13 mbar = 0.1 Torr) 2 h bei 140°C getrocknet und in Diethylether (100 ml) unter Eisbadkühlung gelöst. Die Zinkbromid-Lösung wurde auf 0°C abgekühlt und die Neophylmagnesiumchlorid-Lösung mittels einer Umdrucknadel langsam zugegeben. Es wurde auf Raumtemperatur erwärmt und 14 h nachgerührt. Die ausgefallenen Salze (MgHal₂) wurden durch Filtration über eine trockene, Argon gespülte Umkehrfritte (Porengröße D3) abgetrennt und der Rückstand mit Diethylether (zweimal 40 ml) gewaschen. Anschließend wurde das Lösungsmittel abdestillert und der erhaltene Rückstand in einen 100 ml Rundkolben mit Stickstoffansatz mit einer Umdrucknadel überführt. Zur Entfernung von Lösungsmittelresten wurde 1 h im Vakuum (0.13 mbar = 0.1 Torr) getrocknet. Das Rohprodukt wurde durch Umkondensieren über eine Etherbrücke im Vakuum (1.7 • 10⁻⁴ mbar) bei ca. 150°C (Heißluftfön) gereinigt. Das Produkt Dineophylzink wurde in 61% Ausbeute (14.6 g, 45.9 mmol) isoliert.

### Synthese des funktionalisierten Dialkylzinkreagenzes 4-Pivaloxybutyl-neophylzink:

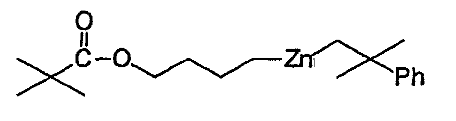

In einem Stickstoffkolben wurde Bis-(4-Pivaloxybutyl)zink, welches ausgehend von 4-Iodobutylpivalat (1.56 g, 5.5 mmol) aus der Iod - Zink - Austauschreaktion in 89% Ausbeute erhalten wurde (das entspricht 1.22 Äquivalenten bezogen auf den Aldehyd), in 2 ml Diethylether gelöst. Zu dieser Lösung wurde Dineophylzink (0.82 g, 2.5 mmol, 1.25 Äquivalente) bei Raumtemperatur zugegeben.

### Enantioselektive Addition von 4-Pivaloxybutyl-neophylzink an Benzaldehyd, Synthese von (S)-5-Hydroxy-5-phenylpentylpivalat:

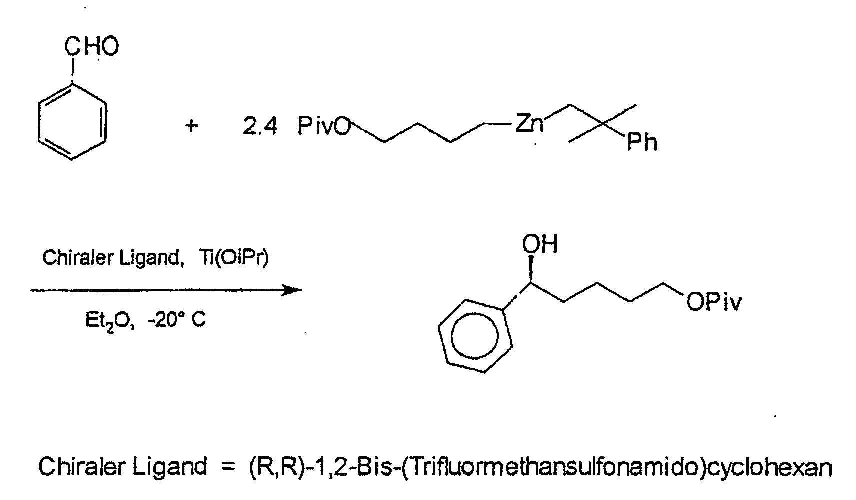

In einem ausgeheizten, Argon gefluteten Rundkolben mit Stickstoffhahn wurde (R,R)-1,2-Bis(Trifluormethansulfonamido)cyclohexan (61 mg, 0.16 mmol, 8 mol%), Titantetraisopropylat (0.36 ml, 1.2 mmol, 0.6 Äquivalente), in 3 ml Diethylether gelöst. Die Katalysatorlösung wurde auf -20°C abgekühlt. Das gemischte Dialkylzinkreagenz 4-Pivaloxybutyl-neophylzink (2.4 Äquivalente) wurde langsam zur Katalysatorlösung zugetropft. Nach 10 Minuten wurde Benzaldehyd (0.21 g, 2.0 mmol, 1 Äquivalent) unverdünnt zugetropft. Das Reaktionsgemisch wurde 23 Stunden bei -20°C gerührt. Nach Hydrolyse mit 20 ml gesättigter Ammoniumchloridlösung und 5 ml 10%iger wässriger Salzsäurelösung wurde die organische Phase abgetrennt und die wässrige Phase noch dreimal mit je 50 ml Diethylether extrahiert. Die vereinigten organischen Phasen wurden mit 2N Natronlauge gewaschen und über Magnesiumsulfat getrocknet. Nach destillativem Entfernen des Lösungsmittels wurde das Rohprodukt säulenchromatographisch gereinigt. Das Produkt (S)-5-Hydroxy-5-phenylpentylpivalat wurde in einer Ausbeute von 82% und einem Enantiomerenüberschuß von 89% isoliert. Der Enantiomerenüberschuß wurde bestimmt durch chirale HPLC-Analyse, Chiracel OD, Heptan / Isopropanol = 95:5, Fluß 0.6 ml/min.; 23.95 min. Hauptisomer, 26.41 min. Nebenisomer; [α]_{D}²⁵ = -21.9° (c = 2.91, CHCl₃).

### Beispiel 2: Synthese von (S)-5-Hydroxy-5-phenylpentylpivalat durch enantioselektive Addition der 4-Pivaloxybutylgruppe an Benzaldehyd unter Zuhilfenahme der entsprechenden Neopentylzinkverbindung

### Synthese des funktionalisierten Dialkylzinkreagenzes 4-Pivaloxybutyl-neopentylzink:

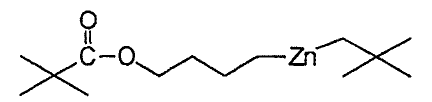

In einem Schlenkkolben wurde Bis-(4-Pivaloxybutyl)zink, welches ausgehend von 4-Iodobutylpivalat (1.56 g, 5.5 mmol) aus der Iod - Zink - Austauschreaktion in 89% Ausbeute erhalten wurde (das entspricht 1.22 Äquivalenten, bezogen auf den Aldehyd), in 2 ml Diethylether gelöst. Zu dieser Lösung wurde Dineopentylzink (0.51 g, 2.5 mmol, 1.25 Äquivalente) bei Raumtemperatur, welches aus der entsprechenden Grignardverbindung hergestellt wurde, zugegeben.

Enantioselektive Addition von 4-Pivaloxybutyl-neopentylzink an Benzaldehyd, Synthese von (S)-5-Hydroxy-5-phenylpentylpivalat:

In einem ausgeheizten, Argon gefluteten Rundkolben mit Stickstoffhahn wurde (R,R)-1,2-Bis(Trifluormethansulfonamido)cyclohexan(61 mg, 0.16 mmol, 8 mol%), Titantetraisopropylat (0.36 ml, 1.2 mmol, 0.6 Äquivalente), in 3 ml Diethylether gelöst. Die Katalysatorlösung wurde auf -20°C abgekühlt. Das gemischte Dialkylzinkreagenz 4-Pivaloxybutyl-neopentylzink (2.4 Äquivalente ) wurde langsam zur Katalysatorlösung zugetropft. Nach 10 Minuten wurde Benzaldehyd (0.21 g, 2.0 mmol, 1 Äquivalent) unverdünnt zugetropft. Das Reaktionsgemisch wurde 23 Stunden bei -20°C gerührt. Nach Hydrolyse mit 20 ml gesättigter Ammoniumchloridlösung und 5 ml 10%iger wässriger Salzsäurelösung wurde die organische Phase abgetrennt und die wässrige Phase noch dreimal mit je 50 ml Diethylether extrahiert. Die vereinigten organischen Phasen wurden mit 2N Natronlauge gewaschen und über Magnesiumsulfat getrocknet. Nach destillativem Entfernen des Lösungsmittels wurde das Rohprodukt säulenchromatographisch gereinigt. Das Produkt (S)-5-Hydroxy-5-phenylpentylpivalat wurde in einer Ausbeute von 82% und einem Enantiomerenüberschuß von 89% isoliert. Der Enantiomerenüberschuß wurde bestimmt durch chirale HPLC-Analyse, Chiracel OD, Heptan / Isopropanol = 95:5, Fluß 0.6 ml/min.; 23.95 min. Hauptisomer, 26.41 min. Nebenisomer; [α]D²⁵ = -22.9° (c = 4.19, CHCl₃).

Aus den Beispielen 1 und 2 ist ersichtlich, daß bei dieser Reaktion der Neophyl- und der Neopentyl-Dummy-Ligand zu identischen Ausbeuten führen.

### Beispiel 3 (Vergleichsbeispiel): Synthese von (S)-1-Phenylethanol durch Addition der Methylgruppe an Benzaldehyd mit Dimethylzink

In einem im Vakuum ausgeheizten, mit Argon gespülten Schlenkgefäß mit Stickstoffansatz wurden (R,R)-1,2-Bis(Trifluormethansulfonamido)cyclohexan (76 mg, 0.19 mmol, 8 mol%), Titantetraisopropylat (0.9 ml, 3.0 mmol, 1.2 Äquivalente), in 3 ml Diethylether gelöst und auf -20°C abgekühlt. Zur Katalysatorlösung wurde Dimethylzink (0.24 ml, 3.5 mmol) langsam zugegeben und 20 min. nachgerührt. Anschließend wurde Benzaldehyd (0.280 g, 2.63 mmol) langsam zugetropft. Nach 16 h wurde mit gesättigter NH₄Cl-Lösung hydrolysiert, der Niederschlag mit 10%iger HCl-Lösung aufgelöst und mit Diethylether (dreimal 50 ml) extrahiert. Nach chromatographischer Reinigung (Petrolether/Et₂O 4:1) wurde das Produkt in 89% Ausbeute (286 mg, 2.34 mmol) als klares Öl erhalten. Die GC-Analyse mit chiraler Säule Daicel Chirasil CD, 120°C, 100 kPa H₂ ergab einen Enantiomerenüberschuß von 23%ee (S-Enantiomer 4.32 min, R-Enantiomer 4.75 min).

### Beispiel 4: Synthese von (S)-1-Phenylethanol durch Addition der Methylgruppe an Benzaldehyd mit Methyl(neophyl)zink

In einem im Vakuum ausgeheizten, mit Argon gespülten Schlenkgefäß mit Stickstoffansatz wurde Dimethylzink (0.15 g, 1.6 mmol) mit Dineophylzink (0.59 g, 1.8 mmol) zur gemischten Zinkverbindung bei Raumtemperatur (RT) umgesetzt. Gleichzeitig wurde in einem weiteren im Vakuum ausgeheizten, mit Argon gespülten Schlenkgefäß mit Stickstoffansatz (R,R)-1,2-Bis(Trifluormethansulfonamido)cyclohexan (61 mg, 0.16 mmol, 8 mol%), Titantetraisopropylat (0.36 ml, 1.2 mmol, 0.6 Äquivalente), in 3 ml Diethylether gelöst und auf -20°C abgekühlt. Zur Katalysatorlösung wurde Methyl(neophyl)zink mittels einer mit Argon gespülten Spritze mit 20 cm Kanüle zugetropft und 20 min nachgerührt. Anschließend wurde Benzaldehyd (287 mg, 2.7 mmol) zur Reaktionslösung langsam zugetropft. Es wurde bei -20°C 16 h nachgerührt. Nach Hydrolyse der Reaktionsmischung mit gesättigter NH₄Cl-Lösung, Auflösen des Niederschlags mit 10%iger HCl-Lösung und Extraktion mit Diethylether (dreimal 50 ml) wurde das erhaltene Rohprodukt chromatographisch gereinigt (Petrolether/Et₂O 4:1). Das Produkt wurde in 76% Ausbeute (250 mg, 2.04 mmol) als klares Öl erhalten. Die GC-Analyse mit chiraler Säule Daicel Chirasil CD, 120°C, 100 kPa H₂ ergab einen Enantiomerenüberschuß von 94%ee. [α]_{D}²⁵= -39.20° (c = 2.32, CHCl₃).

### Beispiel 5: Synthese von (S)-1-Phenylethanol durch Addition der Methylgruppe an Benzaldehyd mit Methyl(neopentyl)zink

In einem im Vakuum ausgeheizten, mit Argon gespülten Schlenkgefäß mit Stickstoffansatz wurde Dimethylzink (0.15 g, 1.6 mmol) mit Dineopentylzink (0.37 g, 1.8 mmol) zur gemischten Zinkverbindung bei RT umgesetzt. Gleichzeitig wurde in einem weiteren im Vakuum ausgeheizten, mit Argon gespülten Schlenkgefäß mit Stickstoffansatz (R,R)-1,2-Bis(Trifluormethansulfonamido)cyclohexan (61 mg, 0.16 mmol, 8 mol%), Titantetraisopropylat (0.36 ml, 1.2 mmol, 0.6 Äquivalente), in 3 ml Diethylether gelöst und auf -20°C abgekühlt. Zur Katalysatorlösung wurde Methyl(neopentyl)zink mittels einer mit Argon gespülten Spritze mit 20 cm Kanüle zugetropft und 20 min nachgerührt. Anschließend wurde Benzaldehyd (217 mg, 2.04 mmol) zur Reaktionslösung langsam zugetropft. Es wurde bei -20°C 16 h nachgerührt. Nach Hydrolyse der Reaktionsmischung mit gesättigter NH₄Cl-Lösung, Auflösen des Niederschlags mit 10%iger HCl-Lösung und Extraktion mit Diethylether (dreimal 50 ml) wurde das erhaltene Rohprodukt chromatographisch gereinigt (Petrolether/Et₂O 4:1). Das Produkt wurde in 92% Ausbeute (229 mg, 1.87 mmol) als klares Öl erhalten. Die GC-Analyse mit chiraler Säule Daicel Chirasil CD, 120°C, 100 kPa H₂ ergab einen Enantiomerenüberschuß von 93%ee. [α]_{D}²⁵= -39.32° (c = 4.92, CHCl₃).

Aus dem Vergleich der Beispiele 3 bis 5 wird deutlich,.daß sich der Enantiomerenüberschuß von 23%ee (bei einer Stoffausbeute von 89%) beim Verfahren nach bisherigem Stand der Technik ohne Dummy-Ligand (Beispiel 3) auf 94%ee (bei einer Stoffausbeute von 76%) bei Verwendung des Neophylrestes als Dummy-Ligand (Beispiel 4), bzw. auf 93%ee (bei einer Stoffausbeute von 92%) bei Verwendung des Neopentylrestes als Dummy-Ligand (Beispiel 5) steigern läßt.

## Patentansprüche

1. Verfahren zur enantioselektiven Alkylierung von Aldehyden mittels Organozinkverbindungen, **dadurch gekennzeichnet, daß** eine funktionalisierte Alkylgruppe (R-FG) nach der allgemeinen Gleichung I 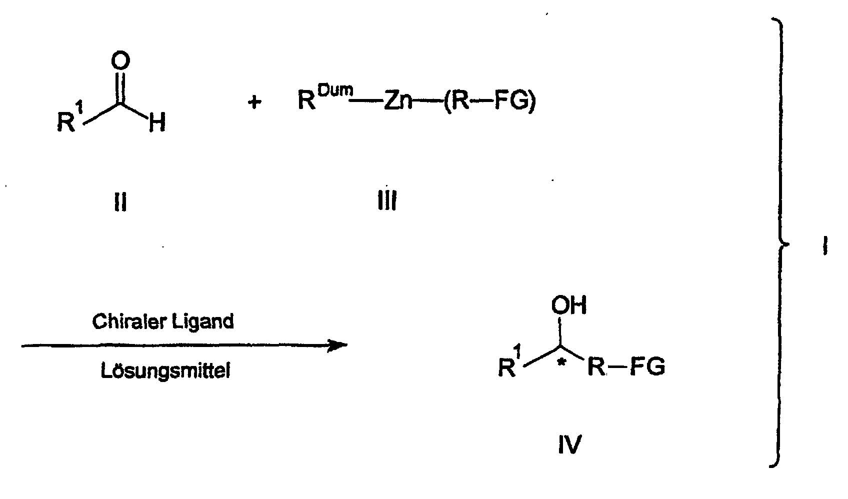 auf einen Aldehyd übertragen wird,
wobei R¹ des Aldehyds II ein Alkyl- oder Arylrest oder ein funktionalisierter Alkyl- oder Arylrest ist,
wobei R^{Dum} der Zinkverbindung III ein organischer Rest der Form -CH₂-C(R²,R³,R⁴) mit
R² = H, Alkyl-, Arylrest
R³ = Alkyl-, Arylrest
R⁴ = Alkyl-, Arylrest ist
oder ein organischer Rest der Form -CH-(CH₃,R⁵) mit R⁵ = Alkylrest ist,
wobei (R-FG) der Zinkverbindung III eine Alkylgruppe oder eine funktionalisierte Alkylgruppe mit einer
Ketocarbonyl-, Ester-, Amin- Carbonsäureamid-,
Nitril-, Cyanat-, Isocyanat-, Ether-, Enolether-,
Halogen-, Aromaten- oder CC-Mehrfachbindungsfunktion ist,
und R^{Dum} ≠ R-FG ist,
wobei das Lösungsmittel ein aliphatischer Kohlenwasserstoff mit 5 bis 12 C-Atomen oder ein aromatischer Kohlenwasserstoff oder ein Ether oder ein chlorierter Kohlenwasserstoff ist,
wobei * in Verbindung IV ein Chiralitätszentrum symbolisiert,
wobei der chirale Ligand in einer Menge von 1 bis 40 mol%, bezogen auf den Aldehyd, in dem Lösungsmittel suspendiert, bzw. gelöst wird, dazu 100 bis 300 mol%, bezogen auf den Aldehyd, der Zinkverbindung III gegeben werden und anschließend der Aldehyd zugegeben wird,
wobei die Reaktion in Gegenwart einer Lewissäure der Form Ti(OR⁶)₄ oder Zr(OR⁶)₄ durchgeführt wird, wobei R⁶ ein Alkyl- oder Arylrest ist, und die Lewissäure in 10 bis 200 mol%, bezogen auf den Aldehyd II, eingesetzt wird, und
wobei die Additionsreaktion in einem Temperaturbereich von -80°C bis 50°C durchgeführt werden.

2. Verfahren nach Anspruch 1, **dadurch gekennzeichnet, daß** die Zinkverbindung III in 160 bis 240 mol%, bezogen auf den Aldehyd II, zugegeben wird.

3. Verfahren nach Anspruch 1 oder 2, **dadurch gekennzeichnet, daß** R^{Dum} der Zinkverbindung III ein Neophylrest oder ein Neopentylrest ist.

4. Verfahren nach den Ansprüchen 1 bis 3, **dadurch gekennzeichnet, daß** (R-FG) der Zinkverbindung III eine Alkylgruppe oder eine funktionalisierte Alkylgruppe mit einer Ester-, Amin- oder Nitrilfunktion ist.

5. Verfahren nach den Ansprüchen 1 bis 4, **dadurch gekennzeichnet, daß** der chirale Ligand in der allgemeinen Gleichung I (R,R)-1,2-Bis-(Trifluormethansulfonamido)cyclohexan (V)
oder (S,S)-1,2-Bis-(Trifluormethansulfonamido)cyclohexan (VI) oder 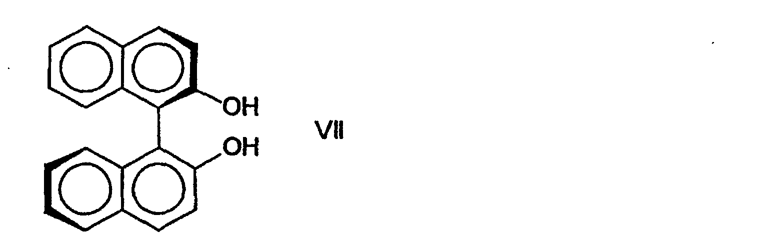 (R)-1-(2-Hydroxy-1-naphtyl)-2-naphtol (VII) oder (S)-1-(2-Hydroxy-1-naphtyl)-2-naphtol (VIII) oder 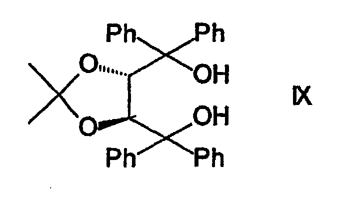 (4S,5S)-2,2-Dimethyl-α,α,α',α'-Tetraphenyl-dioxolan-4,5-dimethanol (IX) oder
(4R,5R)-2,2-Dimethyl-α,α,α',α'-Tetraphenyl-dioxolan-4,5-dimethanol (X) oder 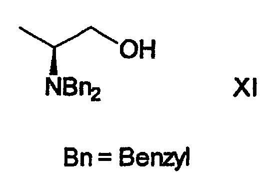 (S)-2-H(Dibenzylamino)propan-1-ol (XI) oder (R)-2-H(Dibenzylamino)propan-1-ol (XII) oder 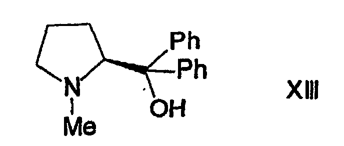 (S)-Diphenyl(1-methylpyrrolidin-2-yl)methanol (XIII) oder (R)-Diphenyl(1-methylpyrrolidin-2-yl)methanol (XIV)
ist und der chirale Ligand in 1 bis 40 mol% bezogen auf den Aldehyd II, zugegeben wird.

6. Verfahren nach Anspruch 5, **dadurch gekennzeichnet, daß** der chirale Ligand in 5 bis 20 mol%, bezogen auf den Aldehyd II, zugegeben wird.

7. Verfahren nach einem der Ansprüche 1 bis 6, **dadurch gekennzeichnet, daß** die Lewissäure in 60 bis 120 mol%, bezogen auf den Aldehyd II, eingesetzt wird.

8. Verfahren nach einem der Ansprüche 1 bis 7, **dadurch gekennzeichnet, daß** R⁶ ein Isopropylrest ist.

9. Alkyl-Zink-Verbindungen der allgemeinen Formel R^{Dum}-Zn-(R-FG), **dadurch gekennzeichnet, daß** R^{Dum} gleich Neophyl (= -CH₂-(C(CH₃)₂Phenyl)) ist und (R-FG) eine Alkylgruppe oder eine funktionalisierte Alkylgruppe mit einer Ketocarbonyl-, Ester-, Amin- Carbonsäureamid-, Nitril-, Cyanat-, Isocyanat-, Ether-, Enolether-, Halogen-, Aromaten- oder CC-Mehrfachbindungsfunktion ist und (R-FG) nicht die Neophylgruppe ist.

10. Alkyl-Zink-Verbindungen nach Anspruch 9 zur Verwendung in einem Verfahren nach den Ansprüchen 1 bis 8.

## Claims

1. Process for the enantioselective alkylation of aldehydes using organozinc compounds, **characterised in that** a functionalised alkyl group (R-FG) according to the general equation I 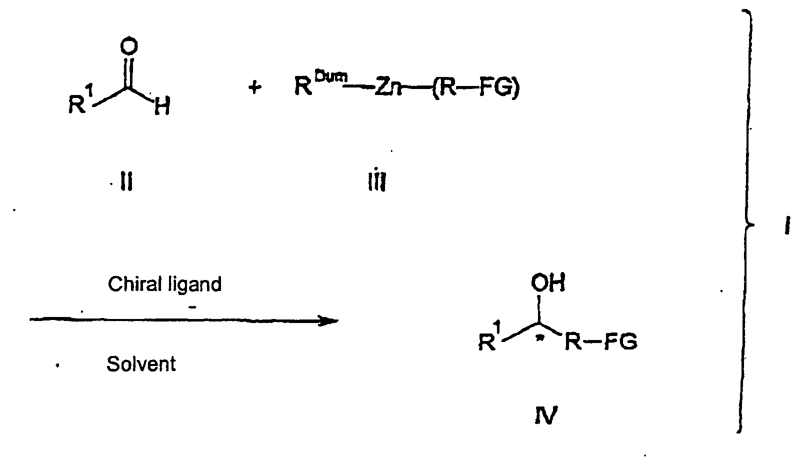 is transferred to an aldehyde,
where R¹ in aldehyde II is an alkyl or aryl radical or a functionalised alkyl or aryl radical,
where R^{Dum} in zinc compound III is an organic radical in the form -CH₂-C(R²,R³,R⁴) where
R² = H, alkyl, aryl radical
R³ = alkyl, aryl radical
R⁴ = alkyl, aryl radical
or an organic radical in the form -CH-(CH₃,R⁵)
where R⁵ = alkyl radical,
where (R-FG) in zinc compound III is an alkyl group or a functionalised alkyl group with a ketocarbonyl, ester, amine, carboxylic acid amide, nitrile, cyanate, isocyanate, ether, enol ether, halogen, aromatic or CC multiple bond function, and R^{Dum} does not equal R-FG,
where the solvent is an aliphatic hydrocarbon with 5 to 12 C atoms or an aromatic hydrocarbon or an ether or a chlorinated hydrocarbon,
where * in compound IV symbolises a chiral centre,
where the chiral ligand is suspended or dissolved in the solvent in a quantity of 1 to 40 mol%, relative to the aldehyde, to which 100 to 300 mol%, relative to the aldehyde, of zinc compound III are added and then the aldehyde is added,
where the reaction is performed in the presence of a Lewis acid in the form Ti(OR⁶)₄ or Zr(OR⁶)₄, where R⁶ is an alkyl or aryl radical, and the Lewis acid is added in 10 to 200 mol%, relative to aldehyde II, and
where the addition reaction is performed in a temperature range from -80°C to 50°C.

2. Process according to claim 1, **characterised in that** zinc compound III is added in 160 to 240 mol%, relative to aldehyde II.

3. Process according to claim 1 or 2, **characterised in that** R^{Dum} in zinc compound III is a neophyl radical or a neopentyl radical.

4. Process according to claims 1 to 3, **characterised in that** (R-FG) in zinc compound III is an alkyl group or a functionalised alkyl group with an ester, amine or nitrile function.

5. Process according to claims 1 to 4, **characterised in that** the chiral ligand in the general equation I is
(R,R)-1,2-bis-(trifluoromethane sulfonamido) cyclohexane (V) or
(S,S)-1,2-bis-(trifluoromethane sulfonamido) cyclohexane (VI) or 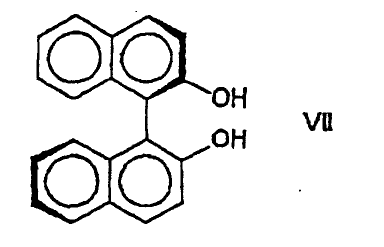 (R)-1-(2-hydroxy-1-naphthyl)-2-naphthol (VII) or
(S)-1-(2-hydroxy-1-naphthyl)-2-naphthol (VIII) or 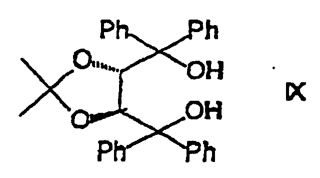 (4S,5S)-2,2-dimethyl-α,α,α',α'-tetraphenyl dioxolane-4,5-dimethanol (IX) or
(4R,5R)-2,2-dimethyl-α,α,α',α'-tetraphenyl dioxolane-4,5-dimethanol (X) or 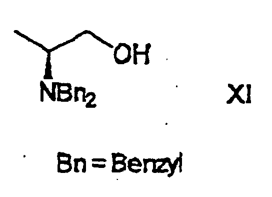 (S)-2-H-(dibenzylamino)propan-1-ol (XI) or
(R) -2-H- (dibenzylamino)propan-1-ol (XII) or 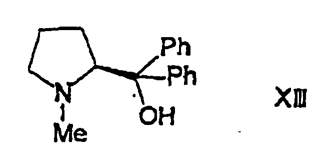 (S)-diphenyl-(1-methyl pyrrolidin-2-yl) methanol (XIII) or
(R)-diphenyl-(1-methyl pyrrolidin-2-yl) methanol (XIV)
and the chiral ligand is added in 1 to 40 mol%, relative to aldehyde II.

6. Process according to claim 5, **characterised in that** the chiral ligand is added in 5 to 20 mol%, relative to aldehyde II.

7. Process according to one of claims 1 to 6, **characterised in that** the Lewis acid is added in 60 to 120 mol%, relative to aldehyde II.

8. Process according to one of claims 1 to 7, **characterised in that** R⁶ is an isopropyl radical.

9. Alkyl-zinc compounds having the general formula R^{Dum}-Zn-(R-FG), **characterised in that** R^{Dum} is equal to neophyl (= -CH₂-(C(CH₃)₂ phenyl)) and (R-FG) is an alkyl group or a functionalised alkyl group with a ketocarbonyl, ester, amine, carboxylic acid amide, nitrile, cyanate, isocyanate, ether, enol ether, halogen, aromatic or CC multiple bond function and (R-FG) is not the neophyl group.

10. Alkyl-zinc compounds according to claim 9 for use in a process according to claims 1 to 8.

## Revendications

1. Procédé d'alkylation énantiosélective d'aldéhydes utilisant des composés organiques de zinc, **caractérisé par le fait qu'**un groupe alkyle fonctionnalisé (R-FG) est transféré sur un aldéhyde selon le schéma réactionnel général I suivant : 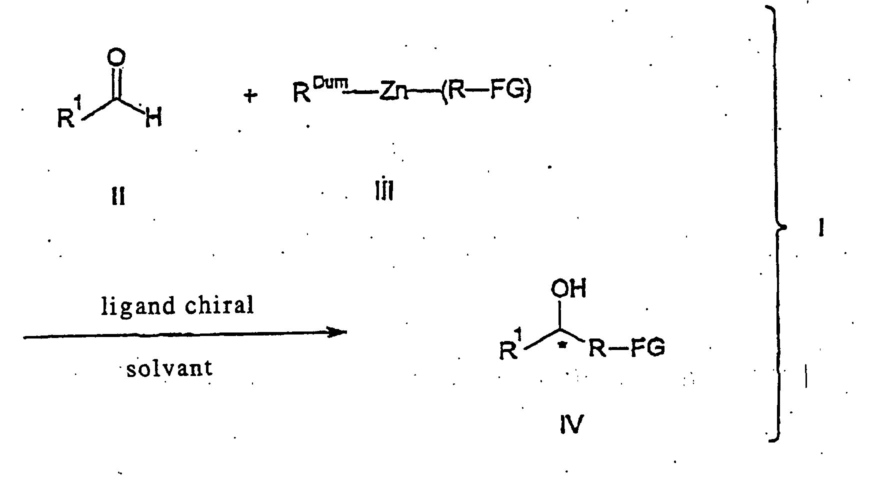 où
le groupe R¹ de l'aldéhyde II est un résidu alkyle ou aryle ou un résidu alkyle ou aryle fonctionnalisé,
le groupe R^{Dum} du composé organique de zine III est un résidu organique de formule
-CH₂-C(R²,R³,R⁴) où R² = H, alkyle ou aryle, R³ = alkyle ou aryle et R4 = alkyle ou aryle, ou un résidu organique de formule
-CH-(CH₃,R⁵) où R⁵ = alkyle,
le résidu (R-FG) du composé de zinc III est un groupe alkyle ou un groupe alkyle fonctionnalisé portant une fonction cétocarbonyle, ester, amine, carbonamide, nitrile, cyanato, isocyanato, éther, énol-éther, halogéno, aromatique ou une liaison carbone-carbone multiple, et
R^{Dum} est différent de R-FG,
le solvant est un hydrocarbure aliphatique comportant de 5 à 12 atomes de carbone ou un hydrocarbure aromatique ou un éther ou un hydrocarbure chloré,
le symbole *, dans le composé de formule IV, désigne un centre chiral,
le ligand chiral est mis en suspension ou est dissous dans le solvant en une quantité comprise entre 1 et 40 % en moles, rapportée à la quantité d'aldéhyde, et l'on y ajoute de 100 à 300 % en moles, rapportés à la quantité d'aldéhyde, de composé de zinc de formule III, puis l'aldéhyde,
la réaction est. réalisée en présence d'un acide de Lewis de formule Ti(OR⁶)₄ ou Zr(OR⁶)₄, où R⁶ représente un résidu alkyle ou aryle, et l'acide de Lewis est utilisé à raison de 10 à 200 % en moles, rapportés à la quantité d'aldéhyde II, et
la réaction d'addition est mise en oeuvre à une température comprise dans l'intervalle allant de -80 °C à 50 °C.

2. Procédé selon la revendication 1, **caractérisé par le fait que** l'on ajoute le composé de zinc III en une quantité comprise entre 160 et 240 % en moles, rapporté à l'aldéhyde II.

3. Procédé selon la revendication 1 ou 2, **caractérisé par le fait que** le résidu R^{Dum} du composé de zinc III est un résidu néophyle ou néopentyle.

4. Procédé selon les revendications 1 à 3, **caractérisé par le fait que** le résidu (R-FG) du composé de zinc III est un groupe alkyle ou un groupe alkyle fonctionnalisé portant une fonction ester, amine ou nitrile.

5. Procédé selon les revendications 1 à 4, **caractérisé par le fait que** le ligand chiral dans le schéma réactionnel I général est le (R,R)-1,2-bis-(trifluorométhanesulfonamido)cyclohexane (V) ou le (S,S)-1,2-bis-(trifluorométhanesulfonamido)cyclohexane (VI) ou 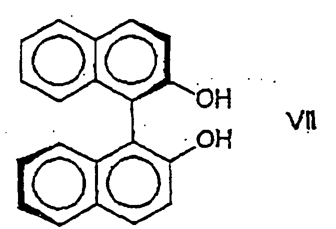 le (R)-1-(2-hydroxy-1-naphtyl)-2-naphtène (VII) ou
le (S)-1-(2-hydroxy-1-naphtyl)-2-naphtène (VIII) ou 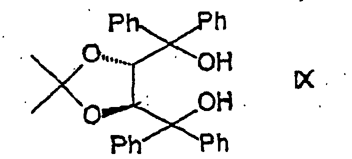 le (4S,5S)-2,2-diméthyl-α,α,α'α'-tétraphényl-dioxolane-4,5-diméthanol (IX) ou
le (4R,5R)-2,2-diméthyl-α,α,α'α'-tétraphényl-dioxolane-4,5-diméthanol (X) ou 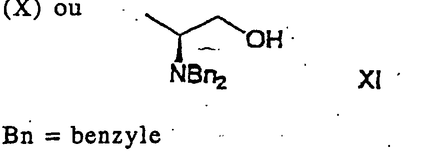 le (S)-2-H(dibenzylamino)propan-1-ol (XI) ou
le (R)-2-H(dibenzylamino)propan-1-ol (XII) ou 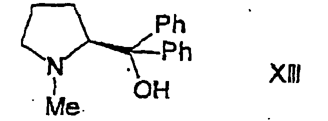 le (S)-diphényl(1-méthylpyrrolidin-2-yl)méthanol (XIII) ou
le (R)-diphényl(1-méthylpyrrolidin-2-yl)méthanol (XIII), et le ligand chiral est ajouté à raison de 1 à 40 % en moles, rapportés à la quantité d'aldéhyde II.

6. Procédé selon la revendication 5, **caractérisé par le fait que** le ligand chiral est ajouté à raison de 5 à 20 % en moles, rapporté à la quantité d'aldéhyde II.

7. Procédé selon l'une des revendications 1 à 6, **caractérisé par le fait que** l'acide de Lewis est utilisé à raison de 60 à 120 % en moles, rapportés la quantité d'aldéhyde II.

8. Procédé selon l'une des revendications 1 à 7, **caractérisé par le fait que** R⁶ représente un résidu isopropylène.

9. Composés alkylés de zinc de formule générale R^{Dum}-Zn-(R-FG), **caractérisés par le fait que** R^{Dum} est un résidu néophyle (= -CH₂-(C(CH₃)₂phényle)) et le résidu (R-FG) est un groupe alkyle ou alkyle fonctionnalisé portant une fonction cétocarbonyle, ester, amine, carbonamide, nitrile, cyanato, isocyanato, éther, énoléther, halogéno, aromatique ou une liaison carbone-carbone multiple et (R-FG) n'est pas un groupe néophyle.

10. Composés alkylés de zinc selon la revendication 9, pour une utilisation dans un procédé selon l'une des revendications 1 à 8.
